# EUROPEAN PATENT SPECIFICATION

(11) **EP 0 699 074 B1**
(45) Date of publication and mention of the grant of the patent: **13.11.2002**
(21) Application number: 94915883.6
(22) Date of filing: 25.04.1994
(51) Int. Cl.: A61K 38/00

(54) **THROMBIN INHIBITORS**
THROMBIN-INHIBITOREN
INHIBITEURS DE THROMBINE

(30) Priority: 30.04.1993 US 55611
(43) Date of publication of application: 06.03.1996
(73) Proprietor: Merck & Co., Inc., Rahway New Jersey 07065-0900 (US)
(72) Inventor: VEBER, Daniel F., Ambler, PA 19002 (US); LEWIS, S. Dale, Lansdale, PA 19446 (US); SHAFER, Jules A., Gwynedd Valley, PA 19437 (US); FENG, Dong-Mei, Harleysville, PA 19438 (US); NUTT, Ruth F., San Diego, CA 92122 (US); BRADY, Stephen, F., Philadelphia, PA 19118 (US)
(74) Representative: Cole, William Gwyn
(86) International application number: US9404539
(87) International publication number: WO94025051

(56) References cited:
- EP-A- 0 019 589
- WO-A-92/07869
- US-A- 4 346 078
- US-A- 4 703 036
- R.F. Nutt et al. 'Novel conformationally constrained amino acids as Lysine-9 substitutions in somatostatin analogs' XP002051191

## Description

### BACKGROUND OF THE INVENTION

Thrombin is a serine protease present in blood plasma in the form of a precursor, prothrombin. Thrombin plays a central role in the mechanism of blood coagulation by converting the solution plasma protein, fibrinogen, into insoluble fibrin.

Edwards et al. *J. Amer. Chem. Soc.* (1992) vol. 114, pp. 1854-63, describes peptidyl α-ketobenzoxazoles which are reversible inhibitors of the serine proteases human leukocyte elastase and porcine pancreatic elastase.

European Publication 363 284 describes analogs of peptidase substrates in which the nitrogen atom of the scissile amide group of the substrate peptide has been replaced by hydrogen or a substituted carbonyl moiety.

Australian Publication 86245677 also describes peptidase inhibitors having an activated electrophilic ketone moiety such as fluoromethylene ketone or α-keto carboxyl derivatives.

Thrombin inhibitors described in prior publications contain sidechains of arginine and lysine. These structures show low selectivity for thrombin over other trypsin-like enzymes. Some of them show toxicity of hypotension and liver toxicity.

Compounds of the invention replace arginine and lysine with aminocyclohexyl moieties. These compounds show selectivity for thrombin over other trypsin-like enzymes and have oral bioavailability.

### SUMMARY OF THE INVENTION

The invention comprises 4-substituted cyclohexylamine derivatives which are thrombin catalytic site inhibitors and which are useful as anticoagulants. These compounds show selectivity for thrombin over trypsin and other trypsin-like enzymes and have oral bioavailability. Trypsin-like enzymes (such as trypsin, thrombin, factor xa, kallikrein, plasmin, urokinase, and plasminogen activator) are serine dependent enzymes that catalyze hydrolysis at arginyl and lysyl peptide bonds.

The invention includes a composition for inhibiting loss of blood platelets, inhibiting formation of blood platelet aggregates, inhibiting formation of fibrin, inhibiting thrombus formation, and inhibiting embolus formation in a mammal, comprising a compound of the invention in a pharmaceutically acceptable carrier. These compositions may optionally include anticoagulants, antiplatelet agents, and thrombolytic agents. The compositions can be added to blood, blood products, or mammalian organs in order to effect the desired inhibitions.

The invention also includes a composition for preventing or treating unstable angina, refractory angina, myocardial infarction, transient ischemic attacks, atrial fibrillation, thrombotic stroke, embolic stroke, deep vein thrombosis, disseminated intravascular coagulation, and reocclusion or restenosis of recanalized vessels, in a mammal, comprising a compound of the invention in a pharmaceutically acceptable carrier. These compositions may optionally include anticoagulants, antiplatelet agents, and thrombolytic agents.

The invention also includes a method for reducing the thrombogenicity of a surface in a mammal by attaching to the surface, either covalently or noncovalently, a compound of the invention.

### DETAILED DESCRIPTION OF THE INVENTION

The invention includes compounds of the formula 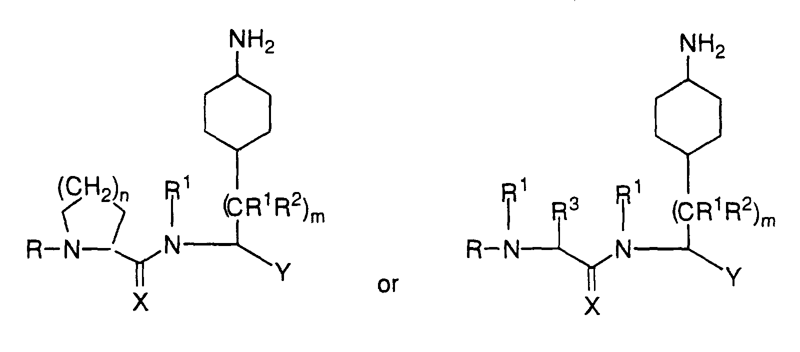 wherein:
m = 0 or 1 ;
n = 0, 1, or 2;
X = O or H₂;
R = arylsulfonyl, aminoacyl, acylaminoacyl, N-C₁₋₃alkyl aminoacyl, acyl-N-C₁₋₃alkylaminoacyl, arylacyl, arylC₁₋₃alkanoyl, hydroxyacyl, aryloxycarbonyl, C₁₋₃alkyloxycarbonyl, or 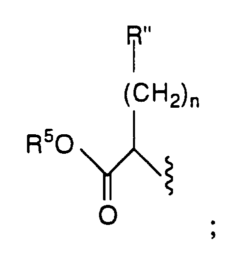
R" = aryl, heteroaryl, C₅₋₁₁carbomonocyclic, or C₅₋₁₁carbobicyclic;
R¹ = H or CH₃;
R² = H or CH₃;
R³ = H, C₁₋₃alkyl, hydroxyC₁₋₃alkyl, carboxyC₁₋₃alkyl, aminoC₁₋₃alkyl, guanidoC₁₋₃alkyl, aryl or substituted aryl, arylmethyl, C₃₋₈ cycloalkylmethyl, or C₃₋₈ cycloalkyl;
Y = CHO, COCF₃, BO₂R⁷R⁸, CO₂R⁴, COOH, CONR⁵R⁶, COCO₂R⁴, COCO₂H, COCO-Q, or CO-W,
   wherein
   Q = a natural amino acid, cyclohexyl amino acid, NR⁵R⁶, or 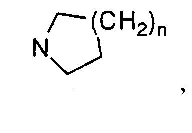 or derivative thereof; and
   W = 5-10 membered heterocyclic groups or substituted heterocyclic groups including, for example, tetrazole, furan, oxazole, benzoxazole, and imidazole;
R⁴ = C₁₋₃alkyl or arylC₁₋₃alkyl;
R⁵ = H, C₁₋₃alkyl or arylC₁₋₃alkyl;
R⁶ = H, C₁₋₃alkyl or arylC₁₋₃alkyl;
R⁷ = H, C₁₋₃alkyl or arylC₁₋₃alkyl; and
R⁸ = H, C₁₋₃alkyl or arylC₁₋₃alkyl,
and pharmaceutically acceptable salts thereof.

Preferably, compounds of the invention have the formula 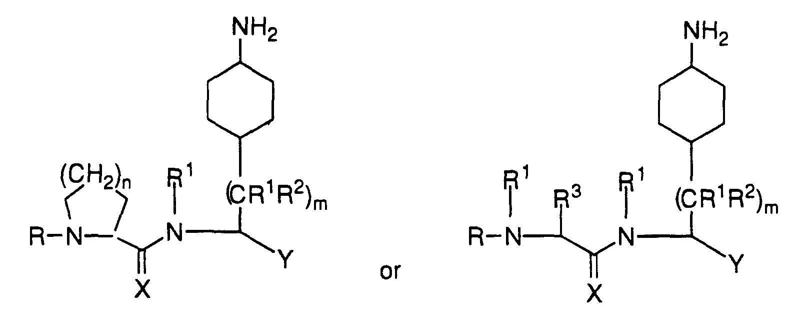 wherein:
n = 0, 1, or 2;
m = 0 or 1;
R = D-phenylalanine, D-Nal1, D-Nal2, D-cyclohexylalanine, D-tyrosine, β-3-benzothienyl-D-alanine, or D-3,4-C12-phenylalanine, or N-methyl derivatives thereof, or 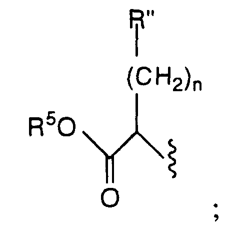
R¹ = H;
R³ = H or cyclohexyl;
X = O or H₂; and
Y = CONH₂, COCO₂H, COCONHCH₃, COCONHCH₂Ph, 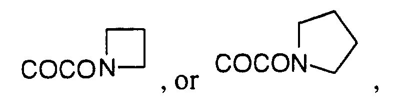
and pharmaceutically acceptable salts thereof.

More preferably, compounds of the invention have the formula 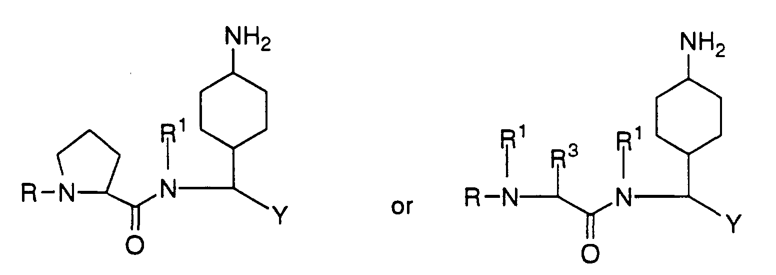 wherein:
R = Nmethyl-D-phenylalanine, Nmethyl-2-naphthyl-D-alanine, Nmethyl- 1-naphthyl-D-alanine, Nmethyl-D-cyclohexylalanine, Nmethyl-D-3,4-Cl₂-D-phenylalanine, or 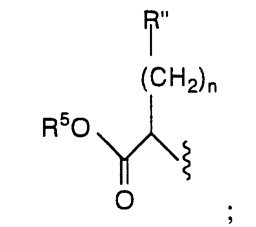
R¹ = H;
R³ = H, cyclohexyl; and
Y = CONH₂, COCO₂H, COCONHCH_{3,} 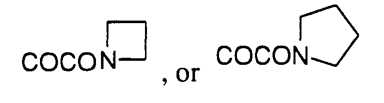
and pharmaceutically acceptable salts thereof.

Preferred embodiments of the invention are shown in the following table.

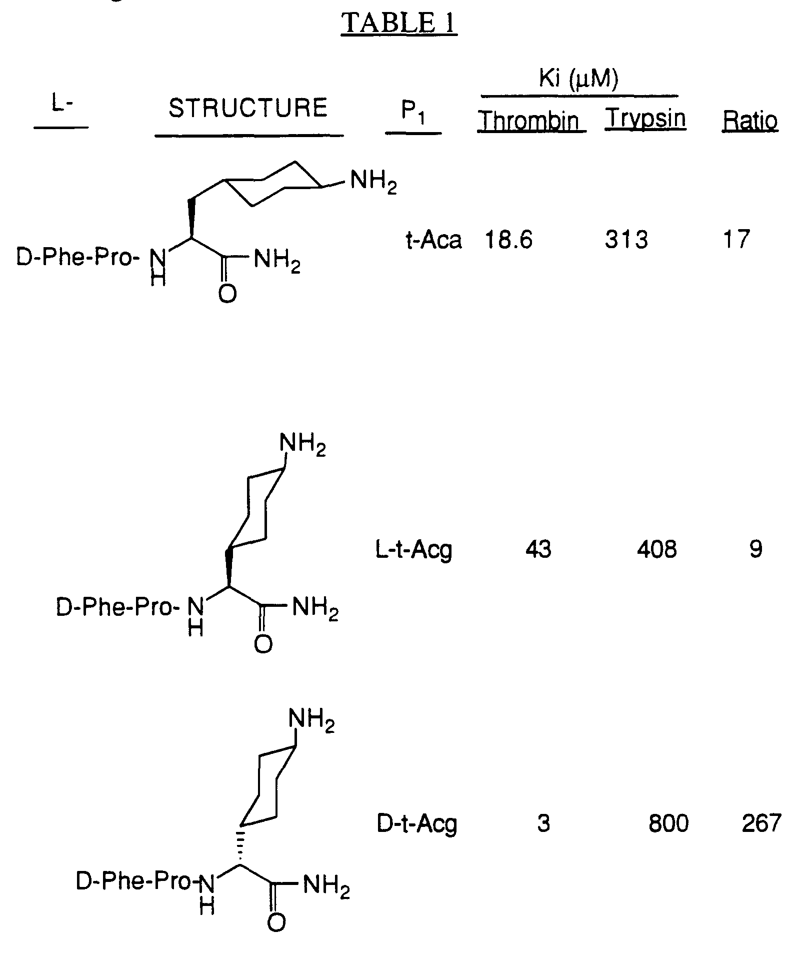

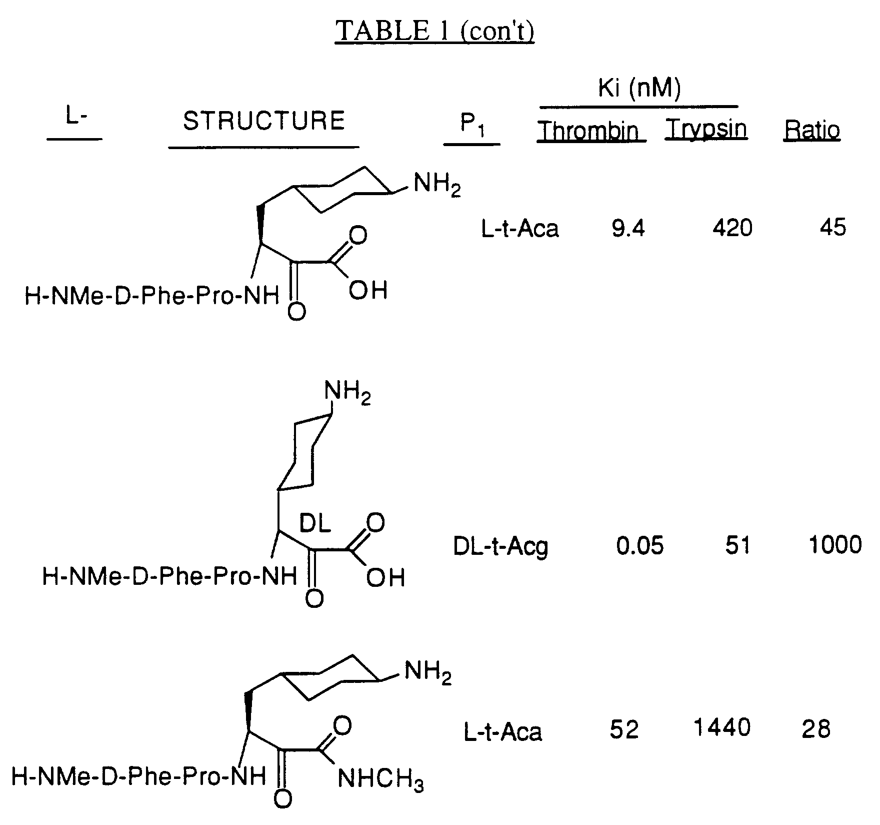

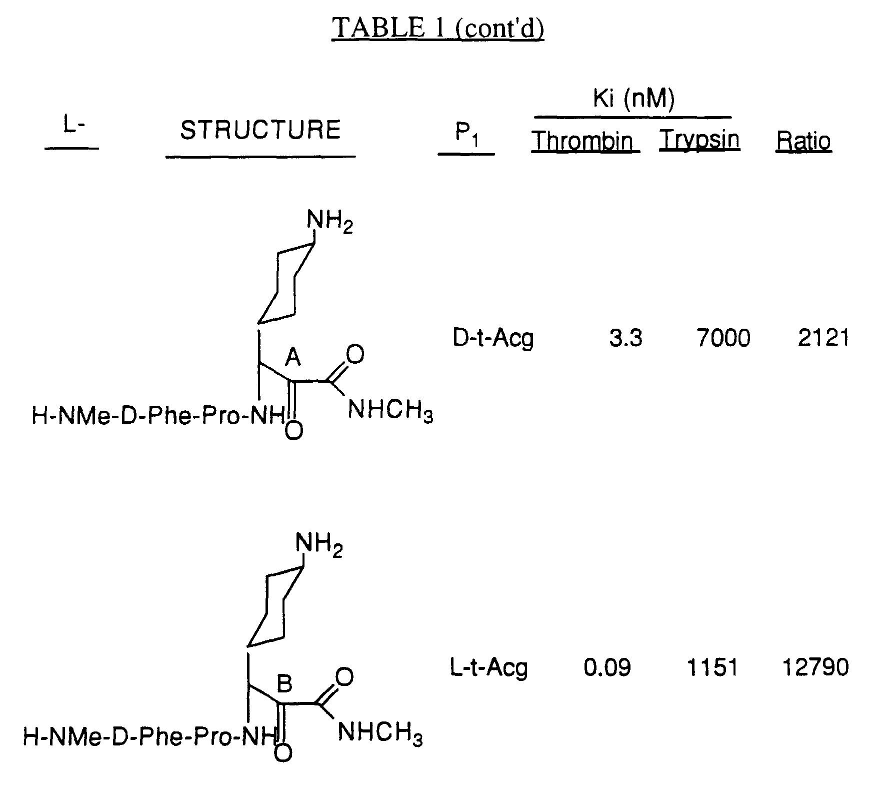

### Assay for determining proteinase inhibition

Assays of human α-thrombin and bovine trypsin were performed at 25°C in 0.05 M TRIS buffer pH 7.4, 0.15 M NaCI, 0.1% PEG. Trypsin assays also contained 1 mM CaCl₂.

In assays wherein rates of hydrolysis of a *p*-nitroanilide (pna) substrate were determined, a Thermomax 96-well plate reader was used was used to measure (at 405 nm) the time dependent appearance of *p*-nitroaniline. sar-PR-pna was used to assay human α-thrombin (Kₘ=125 µM) and bovine trypsin (Kₘ=125 µM). *p*-Nitroanilide substrate concentration was determined from measurements of absorbance at 342 nm using an extinction coefficient of 8270 cm⁻¹M⁻¹.

In certain studies with potent inhibitors (Kᵢ < 10 nM) where the degree of inhibition of thrombin was high, a more sensitive activity assay was employed. In this assay the rate of thrombin catalyzed hydrolysis of the fluorogenic substrate Z-GPR-afc (Kₘ=27 µM) was determined from the increase in fluorescence at 500 nm (excitation at 400 nm) associated with production of 7-amino-4-trifluoromethyl coumarin. Concentrations of stock solutions of Z-GPR-afc were determined from measurements of absorbance at 380 nm of the 7-amino-4-trifluoromethyl coumarin produced upon complete hydrolysis of an aliquot of the stock solution by thrombin.

Activity assays were performed by diluting a stock solution of substrate at least tenfold to a final concentration ≤ 0.1 Kₘ into a solution containing enzyme or enzyme equilibrated with inhibitor. Times required to achieve equilibration between enzyme and inhibitor were determined in control experiments. Initial velocities of product formation in the absence (Vₒ) or presence of inhibitor (Vᵢ) were measured. Assuming competitive inhibition, and that unity is negligible compared Kₘ/[S], [I]/e, and [I]/e (where [S], [I], and e respectively represent the total concentrations, of substrate, inhibitor and enzyme), the equilibrium constant (Kᵢ) for dissociation of the inhibitor from the enzyme can be obtained from the dependence of Vₒ/Vᵢ on [I] shown in equation 1.

Vₒ/Vᵢ = 1 + [I]/Kᵢ (I)

The activities shown by this assay indicate that the compounds of the invention are therapeutically useful for treating various conditions in patients suffering from unstable angina, refractory angina, myocardial infarction, transient ischemic attacks, atrial fibrillation, thrombotic stroke, embolic stroke, deep vein thrombosis, disseminated intravascular coagulation, and reocclusion or restenosis of recanalized vessels. Formulation and administration procedures are described following the Examples section.

The nomenclature used to describe the peptide compounds of the invention follows the conventional practice where the N-terminal amino group is assumed to be to the left and the carboxy group to the right of each amino acid residue in the peptide. In the formulas representing selected specific embodiments of the present invention, the amino- and carboxy-terminal groups, although often not specifically shown, will be understood to be in the form they would assume at physiological pH values, unless otherwise specified. Thus, the N-terminal H⁺₂ and C-terminal O⁻ at physiological pH are understood to be present though not necessarily specified and shown, either in specific examples or in generic formulas. Free functional groups on the side chains of the amino acid residues can also be modified by amidation, acylation or other substitution, which can, for example, change the solubility of the compounds without affecting their activity.

In the peptides shown, each residue, where appropriate, is represented by its three letter designation, corresponding to the trivial name of the amino acid. In addition to the abbreviations commonly used to represent natural amino acids, the abbreviation for naphthylalanine, Nal, is also used, e.g. D-Nal1, D-Nal2. Nail refers to the amino acid where the beta carbon of alanine is attached to the 1-naphthyl position, and Nal2 refers to the amino acid where the beta carbon of alanine is attached to the 2-naphthyl position.

In the specific peptides shown in the present application, the L-form of any amino acid residue having an optical isomer is intended unless the D-form is expressly indicated.

### EXAMPLE 1

### Boc-NMe-D-Phe-Pro-OBz (1-1):

To a solution of Boc-NMe-D-Phe-OH (7.0 g, 25 mmol) and H-Pro-OBzl·HCl (6.66 g, 27.5 mmol) in 200 ml of DMF was added 4.6 g (30 mmol) of HOBt·H₂O, the pH of the solution was adjusted to 8 (moist narrow pH paper), and EDC (6.47 g, 33.8 mmol) was added with magnetic stirring. After 3.5 hrs. the reaction was quenched by the addition of 50 ml of water. After keeping the mixture at room temperature for 16 hrs, the solvents were evaporated at reduced pressure and the residue was dissolved in EtOAc-H₂O. Aqueous KHSO₄ was added to this two-phase mixture and the layers were separated. The organic layer was extracted with NaHCO₃, saturated NaCl, and dried over MgSO₄. The solvent was evaporated to give product as a white solid which was further purified by chromatography using two columns of 600 g silica gel 60 (E. Merck) each and eluting with EtOAc-hexane (3:7). Fractions containing product were combined to give 11.3 g (97% yield) of 1-1.
TLC: R_{f}=0.65, silica gel, EtOAc-hexane (2:3)

### Boc-NMe-D-Phe-Pro-OH (1-2):

A solution of Boc-NMe-D-Phe-Pro-OBz (1-1) (11.3 g) in 600 ml of 95% EtOH was flushed with N2 three times and 1.8 g of 10% Pd/C was added under N₂. The mixture was evacuated, H₂ was introduced and the reaction mixture was kept under a H₂ atmosphere (balloon filled with H₂) for 50 min. The mixture was purged with N₂, filtered through Celite, and the filtrate was evaporated in vacuo. The viscous oil was flushed several times with CHCl₃ to yield a foamy white solid 1-2 (9.13 g, 100% yield).
TLC: R_{f}=0, silica gel, EtOAc-hexane (2:3); Rf=0.3, CHCl₃-MeOH-H₂O (90-10-1)

### EXAMPLE 2

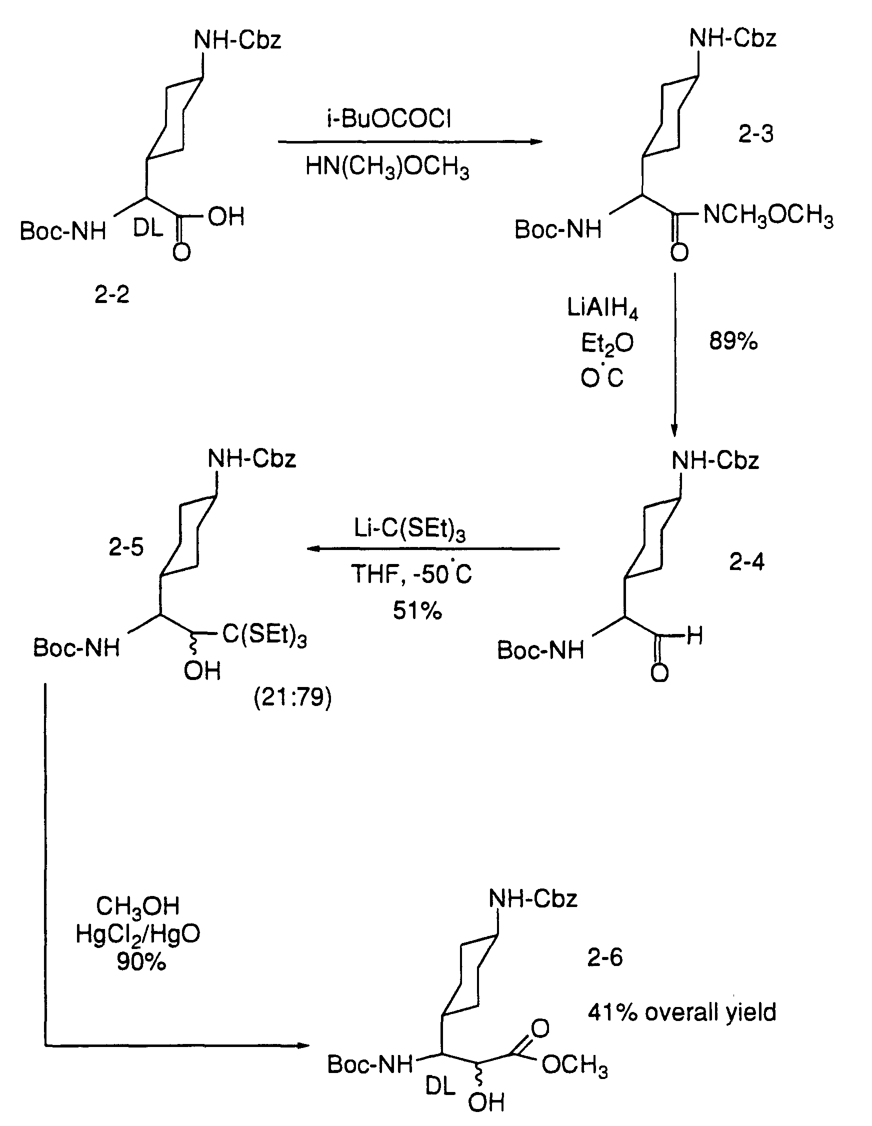

In the following description, "Acg" refers to 4-aminocyclohexylglycine.

### Boc-trans-DL-Acg-OH (2-1):

According to Nutt et al., Peptides: Structure and Function, Proceed. of the 9th Amer. Pept. Symp., eds. C. Deber et al., Pierce Chemical Co. Rockford, IL, 441-444 (1985), 2-1 was obtained. Banfi et al., Syn. Commun. 19 (9&10), 1787-1799 (1989) also describe the synthesis of the unprotected amino acid trans-DL-Acg-OH.

### Boc-trans-DL-Acg(Cbz)-OH (2-2):

2-1 was Cbz-protected according to the procedure in Nutt et al. to give Boc-trans-DL-Acg(Cbz)-OH (2-2).

### Boc-traps-DL-Acg(Cbz)-NMeOMe (2-3):

To a suspension of Boc-trans-Acg(Cbz)-OH (2-2) (794 mg, 1.94 mmol) in CH₂Cl₂ (10 ml) was added N-methyl morpholine (NMM, 0.22 ml). After stirring magnetically for 15 min, all starting material had gone into solution. The temperature was lowered to -15°C and i-butyl chloroformate (0.25 ml, 0.27 g) was added. The mixture was stirred for 10 min and predried HNMeOMe·HCl (0.22 g) was added, followed by NMM (0.15 ml initially, 0.17 ml in increments over the next 40 min.). The reaction mixture was allowed to warm to room temperature and stirred for 18 hrs. Water was added to the reaction and after stirring for 30 min, CH₂Cl₂ was added and the organic layer washed with 1 x KHSO₄ solution, 1 x H₂O, 1 x NaHCO₃ solution, and 2 x 50% saturated NaCl. The organic layer was dried over Na₂SO₄, filtered and evaporated to give 2-3 (746 mg, 85% yield). From the NaHCO₃ extract, starting material (101 mg, 12.7%) was recovered.
TLC: R_{f}=0.75, silica gel, CHCl₃-MeOH-H₂O (95-5-0.5)
HPLC: retention times= 19.43 min, (Vydac C₁₈, gradient of 95%A/B to 5%A/B over 30 min, A=0.1 %TFA-H₂O, B=0.1 %TFA-CH₃CN
NMR: CD₃OD, δ 7.4 (m,5), 5.1 (s.2), 4.5 (br m,1), 3.0 (s, 3), 3.3 (MeOH), 3.2 (s,3), 1.95(m,2), 1.85 (m,1), 1.62(m,2), 1.45 (s,9), 1.1-1.3 (m,4).

### Boc-trans-DL-Acg(Cbz)Ψ[CHO] (2-4):

Into a dried flask equipped with mechanical stirrer, thermometer, and septum was added 616 mg (1.37mmol) of Boc-t-Acg(Cbz)-NMeOMe (2-3) and 18 ml of dry THF. The suspension was cooled to -40°C and the LAH solution (1.78ml of 1M LAH in THF) was added dropwise at a rate to keep the reaction temperature below -30°C. The resultant solution was stirred at -5°C for 50 min, then cooled to -35°C. Ether (15 ml) and an aqueous solution of KHSO₄ were added keeping the temperature at -15°C. The two layer mixture was stirred at room temperature for 30 min, the layers were separated and the aqueous layer was extracted twice with ether. The combined ether layers were extracted once with cold 1N HCl (3 ml), cold 5% NaHCO₃ solution (3 mL), and saturated NaCl solution (3 ml). The ether layer was dried over anhydrous MgSO₄, filtered, and evaporated in vacuo to yield 477 mg (89% yield) of (2-4).
TLC: R_{f}=0.7, silica gel, EtOAc-hexane (3:2)
HPLC: retention time 18.8 min; C₁₈, 100%A to 70% A/B over 30 min, A=0.1 % TF A-H₂O, B=0.1 % TFA-CH₃CN
NMR: CDCl₃, δ 9.62 (s,1,), 7.4 (m,5), 5.1 (m,3), 4.58 (d,1), 4.25 (t,1), 3.42 (m,1), 2.1(m,2), 1.9 (m,2), 1.62(m,1), 1.58(broad, H₂0) 1.42 (s,9), 1.25(m,2), 1.18 (m,2).

### Boc-trans-DL-Acg(Cbz)Ψ[CHOHC(SEt)₃] (2-5):

To a dry 100-ml 3-neck flask equipped with thermometer, magnetic stirrer, and addition funnel was added under N₂ triethylthioorthoformate (1.65 ml, 8.43 mmol) in dry THF (15 ml). The solution was cooled to -65°C and BuLi in THF (2.89 ml, 7.23 mmol) was added dropwise at a rate to keep the temperature below -50°C. The reaction solution was stirred at -60°C for 30 min, and a solution of Boc-trans-Acg(Cbz)Ψ[CHO] (2-4) (470 mg, 1.2 mmol) in THF (4 ml) was added dropwise keeping the reaction solution at -55°C. The addition funnel was washed with two 1-ml portions of THF. After stirring at -40°C for 2 hrs, a solution of NH₄Cl (0.6 g) in H₂O (13.5 ml) and ether (27 ml) were added, and the reaction mixture was allowed to warm to 10°C. The two layers were separated and the aqueous layer was extracted twice with ether. The combined ether layers were washed once with saturated NaCI solution, dried over Na₂SO₄, filtered, and evaporated in vacuo to give an oily residue. Product was isolated by chromatography using 90 g of silica gel (E. Merck 230-400 mesh) and EtOAc-hexane (3:9) as elution solvent. Fractions containing product (Rf=0.3, EtOAc-hexane 3:7) were combined and the solvent was removed by evaporation to give 2-5 (317 mg, 45% yield).
TLC: R_{f}=0.3, silica gel, EtOAc-hexane (3:7)
HPLC: retention times= 25.25 min and 25.72 min, ratio 1:4, (Vydac C₁₈, gradient of 80%A/B to 10%A/B over 30 min, A=0.1%TFA-H₂O, B=0.1%TFA-CH₃CN
retention times= 26.95 min and 27.36 min, ratio 1:4, (Vydac C₁₈, gradient of 95%A/B to 5%A/B over 30 min

### Boc-trans-DL-Acg(Cbz)Ψ[CHOHCO]-OMe (2-6):

To a solution of Boc-trans-Acg(Cbz)Ψ[CHOHC(SEt)3] (2-5) (310 mg, 0.528 mmol) in MeOH-H₂0 (18 ml, 17:1) was added under N₂ with magnetic stirring HgO (183 mg, 0.845 mmol) and HgCl₂ (674 mg, 2.48 mmol). The reaction mixture was stirred at room temperature for 1.5 hr and at 60°C for 30 min. After cooling to room temperature, the reaction mixture was filtered through Celite, the Celite was washed with two 1-ml portions of MeOH, and three 5-ml portions of CH₂Cl₂. To the filtrate was added H₂O (20 ml) and CH₂Cl₂ (10 ml), the layers were separated and the aqueous layer was extracted two times with CH₂Cl₂ (20 ml). To the combined organic layers was added 70% NH₄OAc in H₂0, the CH₂Cl₂ layer was removed from the three-phase mixture, the remaining mixture was extracted two times with CH₂Cl₂, the combined organic layers were washed once with saturated NH₄Cl solution, dried with MgSO₄, filtered, and evaporated in vacuo to give 2-6 (215 mg, 90.4% yield).
TLC: R_{f}=0.4(major), 0.35(minor), silica gel, EtOAc-hexane (1:1)
HPLC: retention times= 18.67 min and 19.02 min, ratio 1:4, (Vydac C₁₈, gradient of 95%A/B to 5%A/B over 30 min, A=0.1%TFA-H₂O, B=0.1%TFA-CH₃CN

### EXAMPLE 3

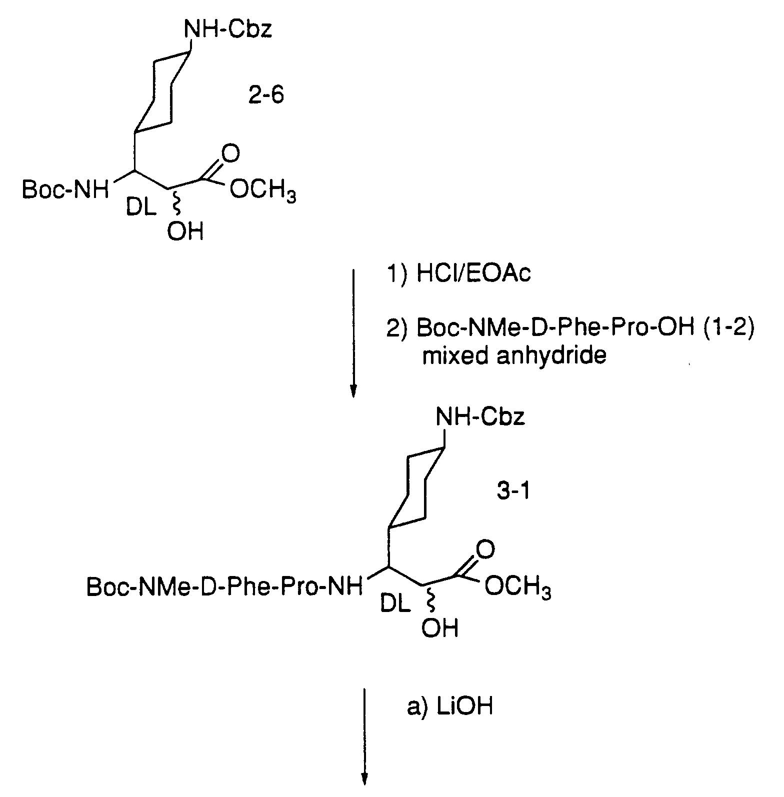

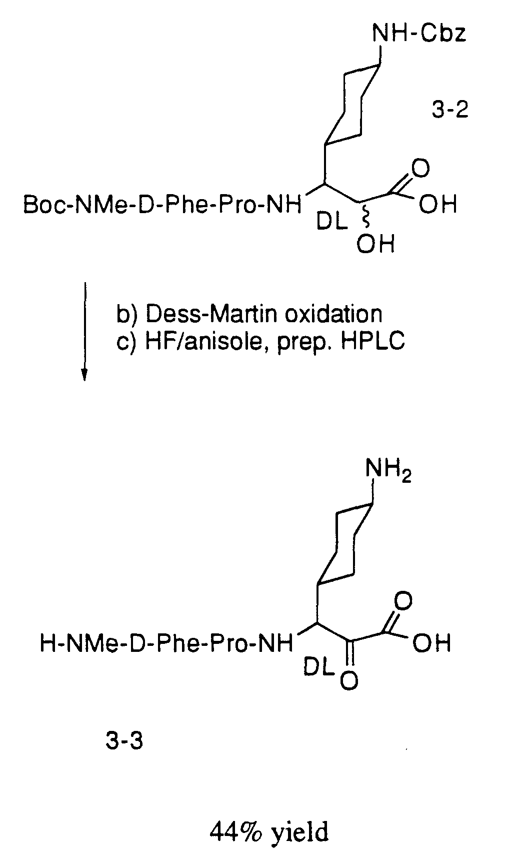

### Boc-NMe-D-Phe-Pro-t-DL-Acg(Cbz)Ψ[CHOHCO]-OMe (3-1):

Preparation of the HCl salt of t-DL-Acg(Cbz)Ψ[CHOHCO]-OMe: A solution of Boc-trans-Acg(Cbz)Ψ[CHOHCO]-OMe (202 mg, 0.448 mmol) in EtOAc (20 ml) was cooled to -25°C under N₂, and gaseous HCl was introduced until saturation, keeping the reaction temperature below -5°C. After 10 min at saturation, the solution was purged with N₂ for 45 min, then excess HCl and solvent were removed by evaporation in vacuo to give the oily HCl salt.

Preparation of Boc-NMe-D-Phe-Pro-t-DL-Acg(Cbz)Ψ[CHOHCO]-OMe (3-1): While the deprotection was carried out, a solution of Boc-NMe-D-Phe-Pro-OH (1-2) (187 mg, 0.493 mmol) in 15 ml of CH₂Cl₂ and 3 ml of EtOAc, to which was added 54 µl of NMM, was cooled to -15°C under N₂ and treated with 64 µl of i-butyl chloroformate. After 10 min at -15°, a solution of the above prepared HCl salt in 4 ml of CH2C12 was added in portions, alternately with NMM (50 µl), followed by NMM addition (35 µl) to bring the pH to 7 (moistened narrow pH paper). The coupling was followed by TLC (85-15-1.5, CHCl₃-MeOH-H₂O) to disappearance of nucleophile. After 2 hrs at -10°C, H₂O was added, the mixture was stirred for 1 hr, the layers separated, and the organic layer washed with 1 x dilute KHSO₄ solution, 1 x H₂O, 1 x NaHCO₃ solution, and 2 x 50% saturated NaCl. The organic layer was dried over Na₂SO₄, filtered and evaporated to dryness. The crude product was purified by chromatography on silica gel, eluting with 99-1-0.1 (CHCl₃-MeOH-H₂O), and the combined fractions containing product were evaporated in vacuo to give 3-1 as a white solid (275 mg, 87% yield).
TLC: R_{f}= 0.45, 0.5 (2 isomers), silica gel, CHCl₃-MeOH-H₂O (95-5-0.5)
HPLC: retention times= 17.2 min and 17.5 min, ratio 46:43, (Vydac C₁₈, gradient of 75%A/B to 20% A/B over 30 min, A=0.1%TFA-H₂O, B=0.1%TFA-CH₃CN

### Boc-NMe-D-Phe-Pro-t-DL-Acg(Cbz)Ψ[CHOHCO]-OH (3-2):

A sample of Boc-NMe-D-Phe-Pro-t-DL-Acg(Cbz)Ψ[CHOHCO]-OMe (3-1) (270 mg, 0.38 mmol) dissolved in 17 ml of 1:1(v/v) THF/H₂O was treated with 2.2 N LiOH (0.22 ml) in portions over 1.5 hrs. keeping the pH at 12-13. After 2.5 hrs., the reaction solution was adjusted to pH 7 with dilute KHSO₄ solution, 50 ml of EtOAc and 25 ml of H₂O were added, and the aqueous layer was further adjusted to pH 2 with KHSO₄ solution. The organic layer was separated and washed twice with 50% saturated NaCl solution, dried over Na₂SO₄, and evaporated in vacuo to give 3-2 (251 mg, 95% yield).
TLC: R_{f}= 0.4, silica gel, upper layer of EtOAc-AcOH-i-octane-H₂O (12-2-2-10)
3-2 was oxidized and deprotected, following procedures described in Example 4 below, to yield 3-3.

### EXAMPLE 4

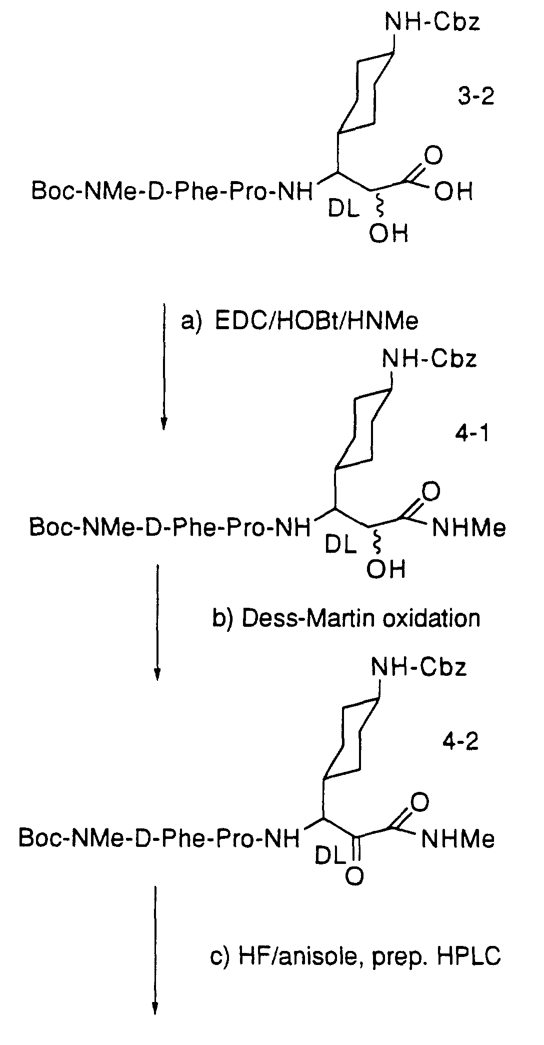

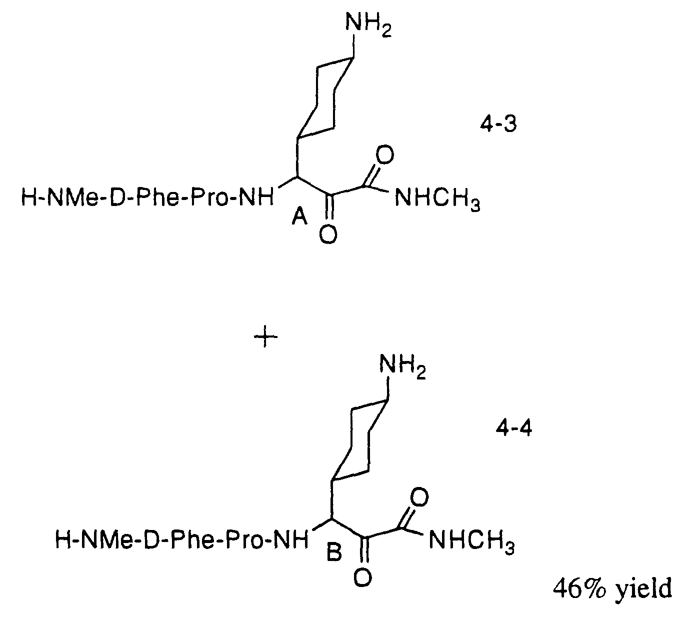

### Boc-NMe-D-Phe-Pro-t-DL-Acg(Cbz)Ψ[CHOHCO]-NHMe (4-1):

To a solution of Boc-NMe-D-Phe-Pro-t-DL-Acg(Cbz)Ψ[CHOHCO]-OH (3-2) (125 mg, 0.18 mmol) in 6.5 ml of dimethyl acetamide was added 34 mg of 1-hydroxybenzotriazole hydrate (HOBt), 35 µl of NMM and 29 mg of methylamine hydrochloride, and the mixture was stirred for 15 min to attain complete dissolution. To this solution was added 1-(3-dimethylaminopropyl)-3-ethylcarbodiimide hydrochloride (EDC, 51 mg) and the resultant mixture was stirred magnetically for 20 hrs. Water (30 ml) and EtOAc (75 ml) were added, the organic layer was washed with dilute KHSO₄ solution, H₂O, NaHCO₃ solution, and 50% saturated NaCl, dried over Na₂SO₄, filtered, and evaporated in vacuo to yield 4-1(117 mg, 92% yield).
TLC: R_{f}= 0.3, silica gel, CHCl₃-MeOH-H₂O (95-5-0.5)

### Boc-NMe-D-Phe-Pro-t-DL-Acg(Cbz)Ψ[COCO]-NHMe (4-2):

To a solution of Boc-NMe-D-Phe-Pro-t-DL-Acg(Cbz)Ψ[CHOHCO]-NHMe (4-1) (115 mg, 0.16 mmol) in 9 ml of CH₂Cl₂ was added with magnetic stirring 0.46 g of periodinane (Dess-Martin reagent). After 1 hr., ether (45 ml) was added, followed by the addition with vigorous stirring of a sodium thiosulfate solution (2.0 g in 25 ml of saturated NaHCO₃). After 5 min. both layers were clear, and the ether layer was separated and washed with dilute NaHCO₃, 50% saturated NaCl, dried over Na₂SO₄, and evaporated in vacuo to give crude keto-amide 4-2.
TLC: R_{f}= 0.50, silica gel, CHCl₃-MeOH-H₂O (95-5-0.5)

### H-NMe-D-Phe-Pro-t-D-AcgΨ[COCO]-NHMe (4-3) and H-NMe-D-Phe-Pro-t-L-AcgΨ[COCO]-NHMe (4-4):

A solution of Boc-NMe-D-Phe-Pro-t-DL-Acg(Cbz)Ψ[COCO]-NHMe (4-2) (125 mg) in 4 ml of CH₂Cl₂ in an HF reactor vessel (Kel-F) was freed of solvent by N₂ purging, and the residue in 1.2 ml of anisole was evacuated to remove residual CH₂Cl₂. Approximately 12 ml of HF was condensed into the reaction vessel at -70°C. After 1.25 hr of stirring at 0-5°, the HF was removed in vacuo, and 20 ml of ether, followed by 20 ml of petroleum ether, were added to the residue. The supernatant was decanted into a sintered glass funnel, and the tacky residue was washed with 3 portions of 1:1 (v/v) ether/ petroleum ether, decanting each as above. After drying in a stream of N₂, the residue in the vessel was dissolved in 25 ml of H₂O, pouring the solution through the above sintered funnel to assure complete recovery of product. The filtrate which contained the two diastereomeric products was charged directly onto the preparative HPLC Waters Prep-Pak C₁₈ column using a gradient elution system of 100%A to 60% A/B over 120 min, at a flow rate of 80 ml/min. The later eluting peak of the two isomers was the more potent thrombin inhibitor and had the L-configuration (4-4) at Acg center.
HPLC: retention times= 16.7 min (D) and 18.5 min (L), Vydac C₁₈, gradient of 100%A to 65%A/B over 30 min, A=0.1%TFA-H₂O, B=0.1%TFA-CH₃CN
FABMS for both isomers: 472 (M+H), 504 (M+MeOH), calcd. mol. wt. = 472

### Thrombin inhibitors - therapeutic uses

Anticoagulant therapy is indicated for the treatment and prevention of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, dogs, cats, rats, and mice.

Thrombin inhibition is useful not only in the anticoagulant therapy of individuals having thrombotic conditions, but is useful whenever inhibition of blood coagulation is required such as to prevent coagulation of stored whole blood and to prevent coagulation in other biological samples for testing or storage. Thus, the thrombin inhibitors can be added to or contacted with any medium containing or suspected of containing thrombin and in which it is desired that blood coagulation be inhibited, e.g. when contacting the mammal's blood with material selected from the group consisting of vascular grafts, stents, orthopedic prothesis, cardiac prosthesis, and extracorporeal circulation systems

The thrombin inhibitors of the invention can be administered in such oral forms as tablets, capsules (each of which includes sustained release or timed release formulations), pills, powders, granules, elixers, tinctures, suspensions, syrups, and emulsions. Likewise, they may be administered in intravenous (bolus or infusion), intraperitoneal, subcutaneous, or intramuscular form, all using forms well known to those of ordinary skill in the pharmaceutical arts. An effective but non-toxic amount of the compound desired can be employed as an anti-aggregation agent.

The thrombin inhibitors can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber or other polymers manufactured by the Dow-Corning Corporation.

The thrombin inhibitors can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The thrombin inhibitors may also be delivered by the use of monoclonal antibodies as individual carriers to which the compound molecules are coupled. The thrombin inhibitors may also be coupled with soluble polymers as targetable drug carriers. Such polymers can include polyvinlypyrrolidone, pyran copolymer, polyhydroxy-propylmethacrylamide-phenol, polyhydroxyethyl-aspartamide-phenol, or polyethyleneoxide-polylysine substituted with palmitoyl residues. Furthermore, the thrombin inhibitors may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The dosage regimen utilizing the thrombin inhibitors is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Oral dosages of the thrombin inhibitors, when used for the indicated effects, will range between about 0.2 mg per kg of body weight per day (mg/kg/day) to about 100 mg/kg/day and preferably 1.0-100 mg/kg/day and most preferably 1-20 mg/kg/day. Intravenously, the most preferred doses will range from about 0.01 to about 10 mg/kg/minute during a constant rate infusion. Advantageously, the thrombin inhibitors may be administered in divided doses of two, three, or four times daily. Furthermore, they can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, or course, be continuous rather than intermittent throughout the dosage regime.

The thrombin inhibitors are typically administered as active ingredients in admixture with suitable pharmaceutical diluents, excipients or carriers (collectively referred to herein as "carrier" materials) suitably selected with respect to the intended form of administration, that is, oral tablets, capsules, elixers, syrups and the like, and consistent with convention pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, distintegrating agents and coloring agents can also be incorporated into the mixture. Suitable binders include starch, gelatin, natural sugars such as glucose or beta-lactose, cornsweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch methyl cellulose, agar, bentonite, xanthan gum and the like.

The thrombin inhibitors can also be co-administered with suitable anti-coagulation agents or thrombolytic agents such as plasminogen activators or streptokinase to achieve synergistic effects in the treatment of various ascular pathologies. For example, thrombin inhibitors enhance the efficiency of tissue plasminogen activator-mediated thrombolytic reperfusion. Thrombin inhibitors may be administered first following thrombus formation, and tissue plasminogen activator or other plasminogen activator is administered thereafter. They may also be combined with heparin, aspirin, or warfarin.

## Claims

1. Compounds of the formula 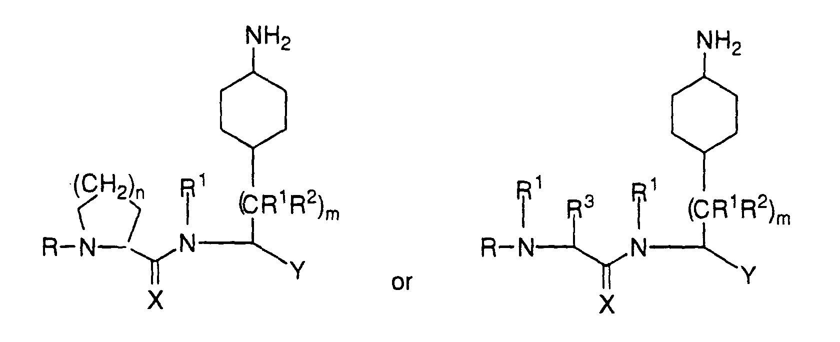 wherein:
m = 0 or 1;
n = 0, 1, or 2;
X = O or H₂;
R = arylsulfonyl, aminoacyl, acylaminoacyl, N-C₁₋₃alkyl aminoacyl, acyl-N-C₁₋₃alkylaminoacyl, arylacyl, arylC₁₋₃alkanoyl, hydroxyacyl, aryloxycarbonyl, C₁₋₃alkyloxycarbonyl, or 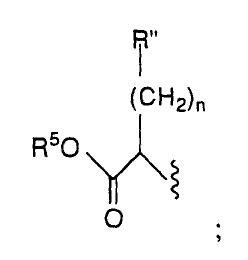
R" = aryl, heteroaryl, C₅₋₁₁carbomonocyclic, or C₅₋₁₁carbobicyclic;
R¹ = H or CH₃;
R² = H or CH₃;
R³ = H, C₁₋₃alkyl, hydroxyC₁₋₃alkyl, carboxyC₁₋₃alkyl, aminoC₁₋₃alkyl, guanidoC₁₋₃alkyl, aryl or substituted aryl, arylmethyl, C₃₋₈ cycloalkylmethyl, or C₃₋₈ cycloalkyl;
Y = CHO, COCF₃, BO₂R⁷R⁸, CO₂R⁴, COOH, CONR⁵R⁶, COCO₂R⁴, COCO₂H, COCO-Q, or CO-W,
wherein
Q = a natural amino acid, cyclohexyl amino acid, NR⁵R⁶, or 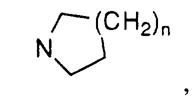 or derivative thereof; and
W = 5-10 membered heterocyclic groups or substituted heterocyclic groups;
R⁴ = C₁₋₃alkyl or arylC₁₋₃alkyl;
R⁵ = H, C₁₋₃alkyl or arylC₁₋₃alkyl;
R⁶ = H, C₁₋₃alkyl or arylC₁₋₃alkyl;
R⁷ = H, C₁₋₃alkyl or arylC₁₋₃alkyl; and
R⁸ = H, C₁₋₃alkyl or arylC₁₋₃alkyl,
and pharmaceutically acceptable salts thereof.

2. Compounds of claim 1 having the formula 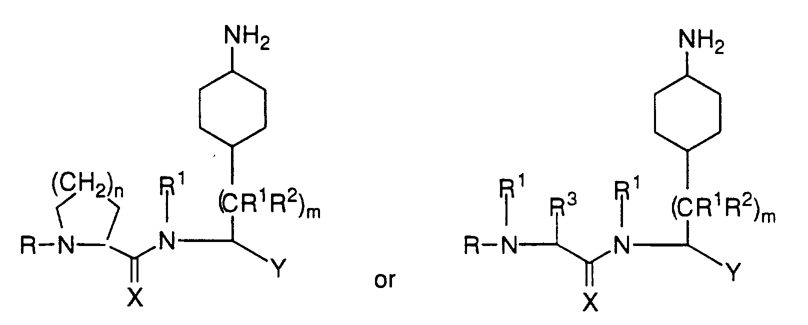 wherein:
n = 0, 1, or 2;
m = 0 or 1;
R = D-phenylalanine, D-Nal1, D-Nal2, D-cyclohexylalanine, D-tyrosine, β-3-benzothienyl-D-alanine, or D-3,4-Cl₂-phenylalanine, or N-methyl derivatives thereof, or 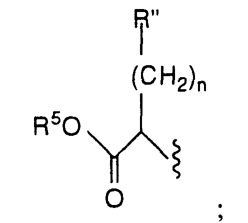
R¹ = H;
R³ = H or cyclohexyl;
X = O or H₂; and
Y = CONH₂, COCO₂H, COCONHCH₃, COCONHCH₂Ph, 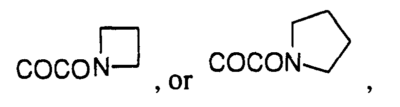
and pharmaceutically acceptable salts thereof.

3. Compounds of claim 2 having the formula: 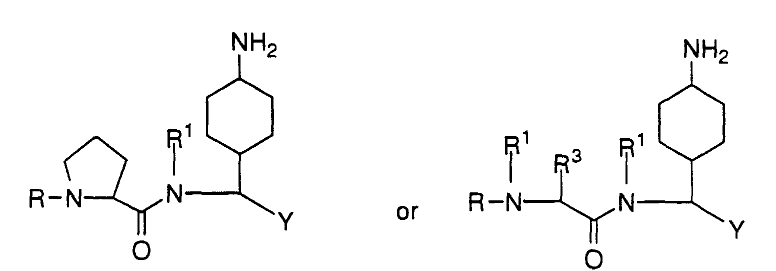 wherein:
R = Nmethyl-D-phenylalanine, Nmethyl-2-naphthyl-D-alanine, Nmethyl-1-naphthyl-D-alanine, Nmethyl-D-cyclohexylalanine, Nmethyl-D-3,4-Cl₂-D-phenylalanine, or 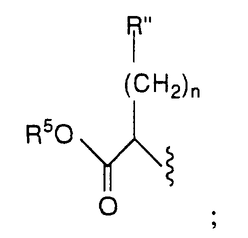
R¹ = H;
R³ = H, cyclohexyl; and
Y = CONH₂, COCO₂H, COCONHCH₃, 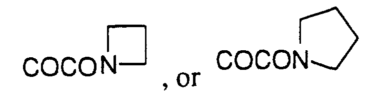
and pharmaceutically acceptable salts thereof.

4. Compounds of claim 3 selected from the group consisting of: 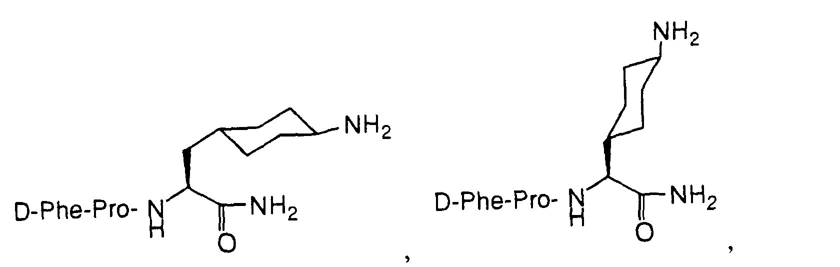 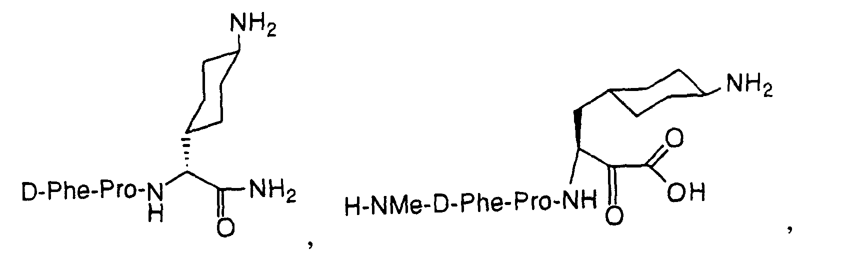 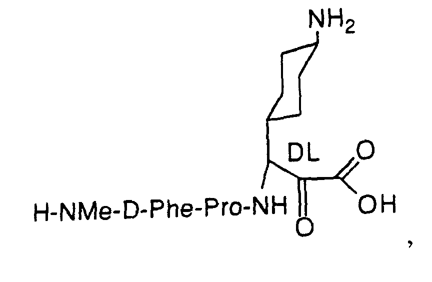 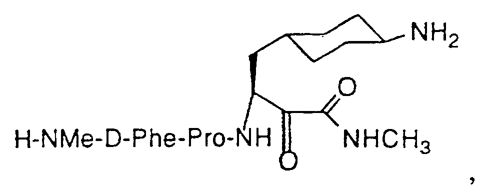 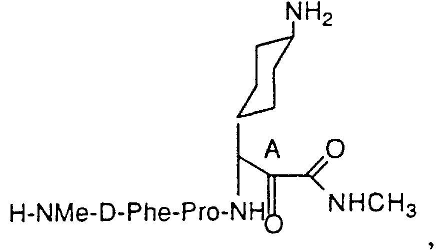 and

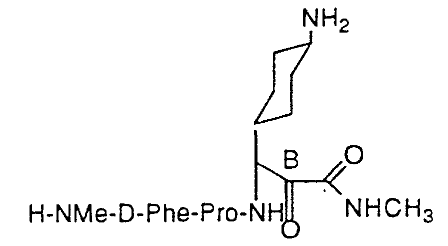

5. A compound of Claim 1 for use in inhibiting thrombin or preventing thrombus or embolus formation in a mammal.

6. A pharmaceutical composition comprising a compound of anyone of Claims 1 to 5 or a pharmaceutically acceptable salt thereof and a pharmaceutically acceptable carrier.

7. Use of a compound of anyone of claims 1 to 5, or a pharmaceutically acceptable salt thereof, in the manufacture of a medicament for use in inhibiting thrombin or preventing thrombus or embolus formation in a mammal.

8. Use of a compound of any one of claims 1 to 5, or a pharmaceutically acceptable salt thereof, for preventing the coagulation of stored whole blood or the coagulation of other biological samples containing thrombin.

## Patentansprüche

1. Verbindungen der Formel 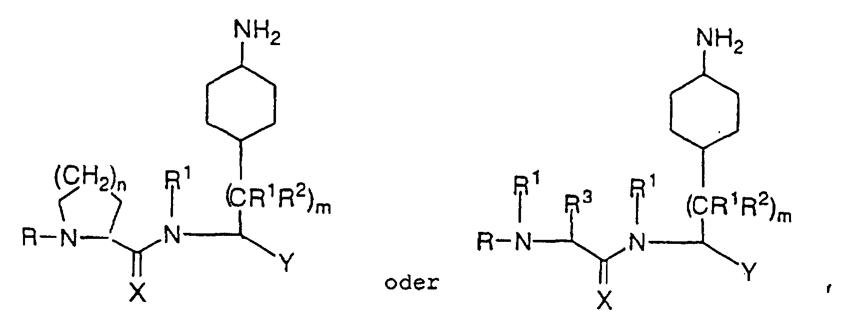 wobei:
m = 0 oder 1,
n = 0, 1 oder 2,
X = O oder H₂,
R = Arylsulfonyl, Aminoacyl, Acylaminoacyl, N-C₁₋₃-Alkylaminoacyl, Acyl-N-C₁₋₃-alkylaminoacyl, Arylacyl, Aryl-C₁₋₃-alkanoyl, Hydroxyacyl, Aryloxycarbonyl, C₁₋₃-Alkyloxycarbonyl oder 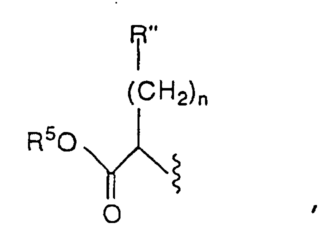
R" = Aryl, Heteroaryl, C₅₋₁₁-carbomonocyclisch oder C₅₋₁₁-carbobicyclisch,
R¹ = H oder CH₃,
R² = H oder CH₃,
R³ = H, C₁₋₃-Alkyl, Hydroxy-C₁₋₃-alkyl, Carboxy-C₁₋₃-alkyl, Amino-C₁₋₃-alkyl, Guanido-C₁₋₃-alkyl, Aryl oder substituiertes Aryl, Arylmethyl, C₃₋₈-Cycloalkylmethyl oder C₃₋₈-Cycloalkyl, Y = CHO, COCF₃, BO₂R⁷R⁸, CO₂R⁴, COOH, CONR⁵R⁶, COCO₂R⁴, COCO₂H, COCO-Q oder CO-W,
wobei
Q = eine natürliche Aminosäure, Cyclohexylaminosäure, NR⁵R⁶ oder 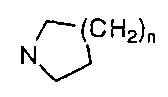 oder ein Derivat davon, und
W = 5-10gliedrige heterocyclische Gruppen oder substituierte heterocyclische Gruppen,
R⁴ = C₁₋₃-Alkyl oder Aryl-C₁₋₃-alkyl,
R⁵ = H, C₁₋₃-Alkyl oder Aryl-C₁₋₃-alkyl,
R⁶ = H, C₁₋₃-Alkyl oder Aryl-C₁₋₃-alkyl,
R⁷ = H, C₁₋₃-Alkyl oder Aryl-C₁₋₃-alkyl und
R⁸ = H, C₁₋₃-Alkyl oder Aryl-C₁₋₃-alkyl,
und pharmazeutische annehmbare Salze davon.

2. Verbindungen nach Anspruch 1 mit der Formel 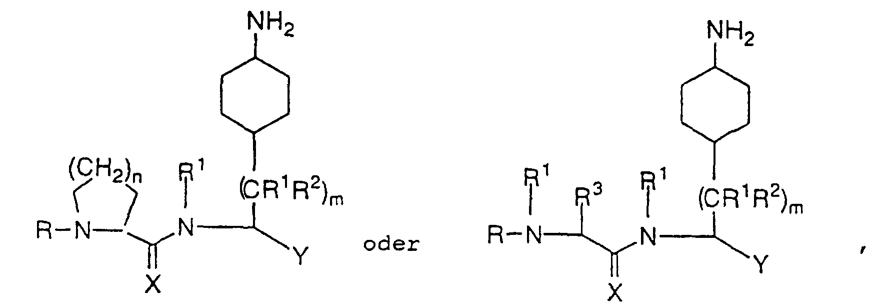 wobei:
n = 0, 1 oder 2,
m = 0 oder 1,
R = D-Phenylalanin, D-Nal1, D-Nal2, D-Cyclohexylalanin, D-Tyrosin, β-3-Benzothienyl-D-alanin oder D-3,4-Cl₂-Phenylalanin oder N-Methylderivate davon oder 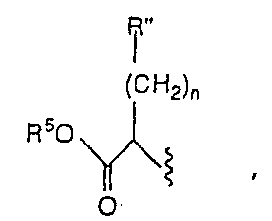
R¹ = H,
R³ = H oder Cyclohexyl,
X = O oder H₂ und
Y = CONH₂, COCO₂H, COCONHCH₃, COCONHCH₂Ph, 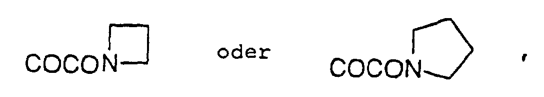
und pharmazeutisch annehmbare Salze davon.

3. Verbindungen nach Anspruch 2 mit der Formel: 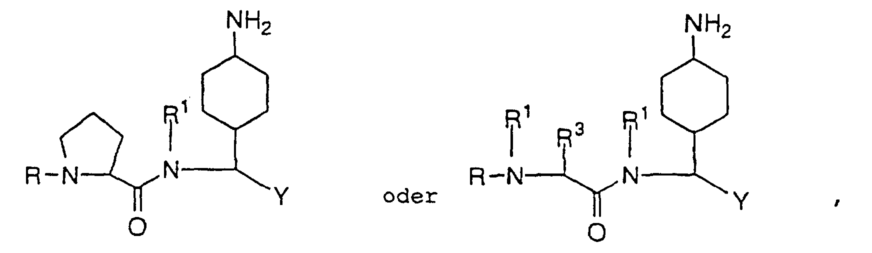 wobei:
R = N-Methyl-D-Phenylalanin, N-Methyl-2-naphthyl-D-alanin, N-Methyl-1-naphthyl-D-alanin, N-Methyl-D-cyclohexylalanin, N-Methyl-D-3,4-Cl₂-D-phenylalanin oder 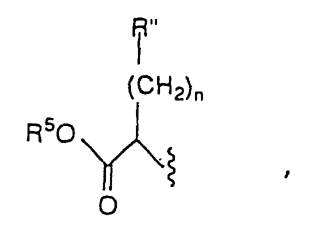
R¹ = H,
R³ = H, Cyclohexyl und
Y = CONH₂, COCO₂H, COCONHCH₃, 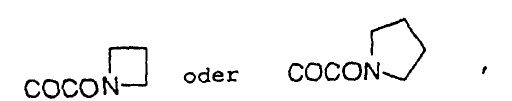
und pharmazeutisch annehmbare Salze davon.

4. Verbindungen nach Anspruch 3, ausgewählt aus der Gruppe, bestehend aus: 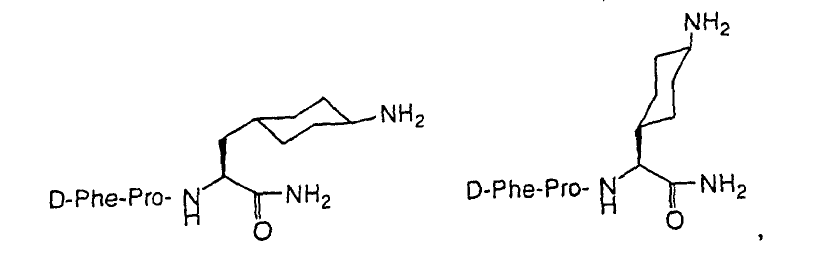 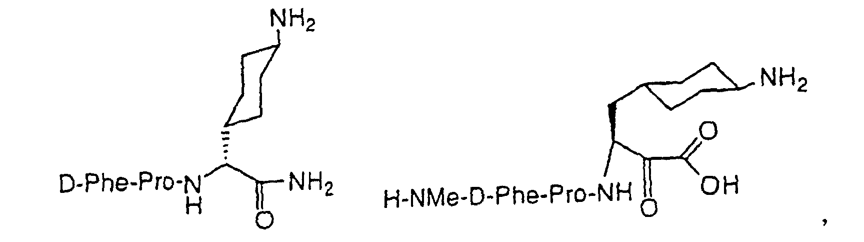 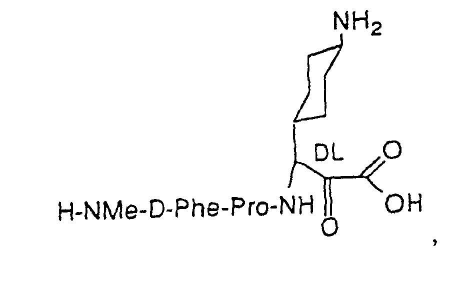 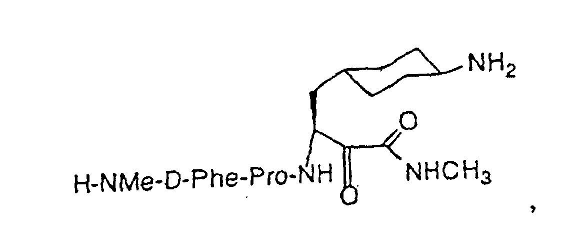 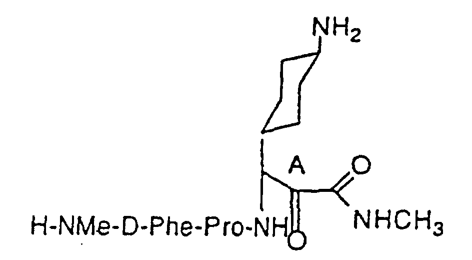 und

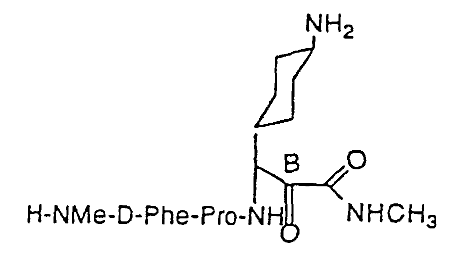

5. Eine Verbindung nach Anspruch 1 zur Verwendung bei der Inhibierung von Thrombin oder Prävention von Thrombus- oder Embolusbildung bei einem Säugetier.

6. Eine pharmazeutische Zusammensetzung, die eine Verbindung nach irgendeinem der Ansprüche 1 bis 5 oder ein pharmazeutisch annehmbares Salz davon und einen pharmazeutisch annehmbaren Träger enthält.

7. Die Verwendung einer Verbindung nach irgendeinem der Ansprüche 1 bis 5 oder eines pharmazeutisch annehmbaren Salzes davon bei der Herstellung eines Medikaments zur Verwendung bei der Inhibierung von Thrombin oder Prävention von Thrombus- oder Embolusbildung bei einem Säugetier.

8. Die Verwendung einer Verbindung nach irgendeinem der Ansprüche 1 bis 5 oder eines pharmazeutisch annehmbaren Salzes davon zur Prävention der Koagulation von gelagertem Vollblut oder der Koagulation anderer biologischer Proben, die Thrombin enthalten.

## Revendications

1. Composés de formule : 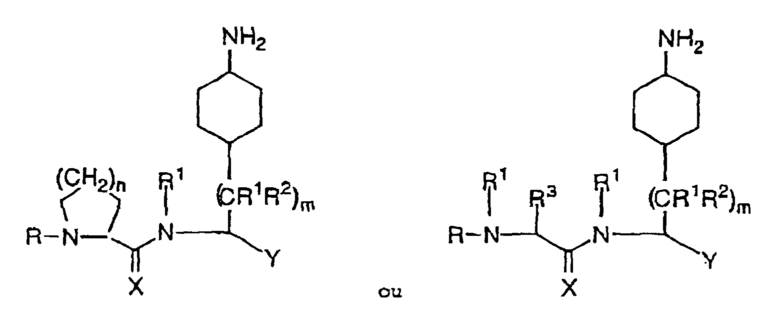 dans laquelle :
m = 0 ou 1 ;
n = 0, 1 ou 2 ;
X = 0 ou H₂ ;
R = un groupe arylsulfonyle, aminoacyle, acylaminoacyle, N-(alkyle en C₁₋₃)aminoacyle, acyl-N-(alkyle en C₁₋₃)aminoacyle, arylacyle, aryl(alcanoyle en C₁₋₃), hydroxyacyle, aryloxycarbonyle, (alkyle en C₁₋₃)-oxycarbonyle, ou 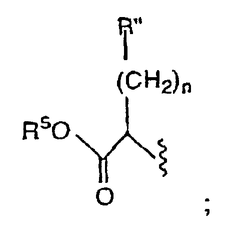
R" = un groupe aryle, hétéroaryle, carbomonocyclique en C₅₋₁₁ ou carbobicyclique en C₅₋₁₁ ;
R¹ = H ou CH₃ ;
R² = H ou CH₃ ;
R³ = H ou un groupe alkyle en C₁₋₃, hydroxyalkyle en C₁₋₃, carboxy(alkyle en C₁₋₃), aminoalkyle en C₁₋₃, guanido(alkyle en C₁₋₃), aryle ou aryle substitué, arylméthyle, (cycloalkyle en C₃₋₈)méthyle ou cycloalkyle en C₃₋₈ ;
Y = CHO, COCF₃, BO₂R⁷R⁸, CO₂R⁴, COOH, CONR⁵R⁶, COCO₂R⁴, COCO₂H, COCO-Q ou CO-W,
où Q = un amino-acide naturel, un cyclohexylamino-acide, NR⁵R⁶ ou 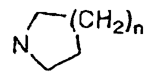 ou un de ses dérivés ; et
W = un groupe hétérocyclique ou hétérocyclique substitué à 5 - 10 chaînons ;
R⁴ = un groupe alkyle en C₁₋₃ ou aryl(alkyle en C₁₋₃) ;
R⁵ = H ou un groupe alkyle en C₁₋₃ ou aryl(alkyle en C₁₋₃) ;
R⁶ = H ou un groupe alkyle en C₁₋₃ ou aryl(alkyle en C₁₋₃) ;
R⁷ = H ou un groupe alkyle en C₁₋₃ ou aryl(alkyle en C₁₋₃) ; et
R⁸ = H ou un groupe alkyle en C₁₋₃ ou aryl(alkyle en C₁₋₃) ;
et leurs sels pharmaceutiquement acceptables.

2. Composés selon la revendication 1, répondant à la formule : 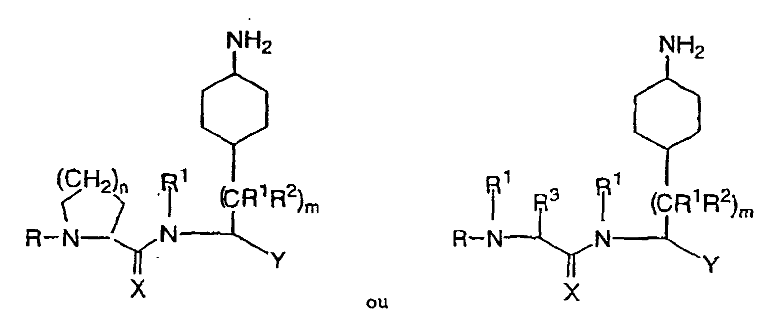 dans laquelle
n = 0, 1 ou 2 ;
m = 0 ou 1 ;
R = D-phénylalanine, D-Nal1, D-Nal2, D-cyclohexylalanine, D-tyrosine, β-3-benzothiényl-D-alanine ou D-3,4-Cl₂-phénylalanine ou leurs dérivés N-méthyle, ou 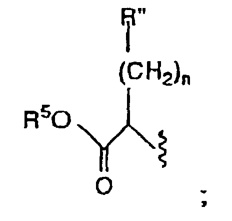
R¹ = H ;
R³ = H ou un groupe cyclohexyle ;
X = O ou H₂ ; et
Y = CONH₂, COCO₂H, COCONHCH₃, COCONHCH₂Ph, 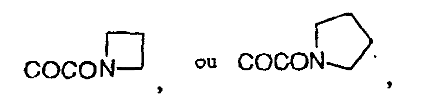
et leurs sels pharmaceutiquement acceptables.

3. Composés selon la revendication 2, répondant à la formule : 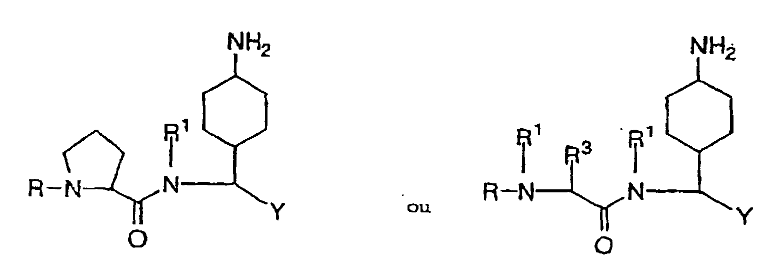 dans laquelle :
R = Nméthyl-D-phénylalanine, Nméthyl-2-naphtyl-D-alanine, Nméthyl-1-naphtyl-D-alanine, Nméthyl-D-cyclohexylalanine, Nméthyl-D-3,4-Cl₂-D-phénylalanine, ou 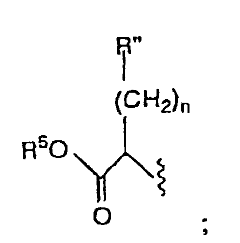
R¹ = H ;
R³ = H, cyclohexyle ; et
Y = CONH₂, COCO₂H, COCONHCH₃, 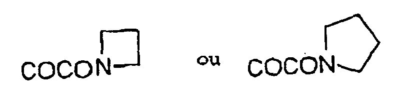
et leurs sels pharmaceutiquement acceptables.

4. Composés selon la revendication 3 choisis dans le groupe comprenant :

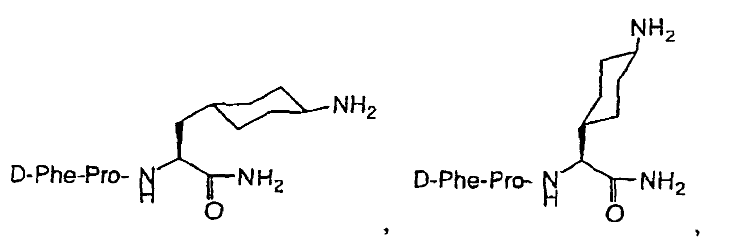

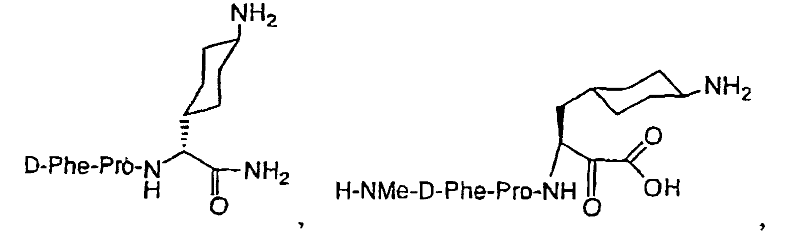

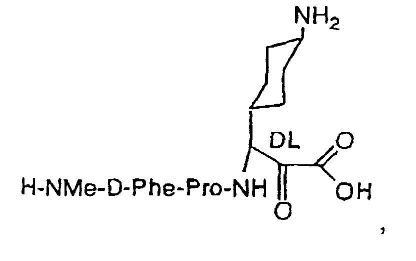

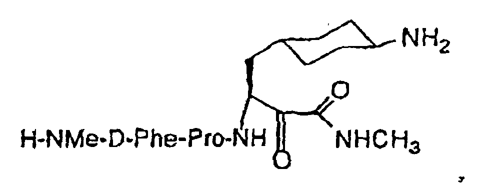

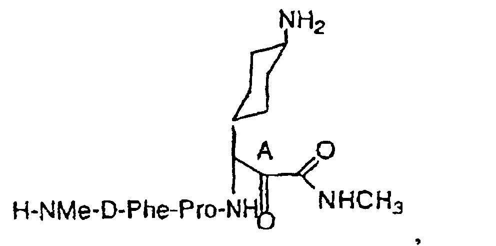

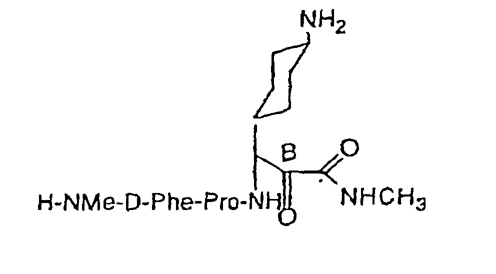

5. Composé selon la revendication 1, à utiliser dans l'inhibition de la thrombine ou la prévention du thrombus ou de la formation d'embole chez un mammifère.

6. Composition pharmaceutique qui comprend un composé selon l'une quelconque des revendications 1 à 5 ou un de ses sels pharmaceutiquement acceptables et un véhicule pharmaceutiquement acceptable.

7. Utilisation d'un composé selon l'une quelconque des revendications 1 à 5, ou d'un de ses sels pharmaceutiquement acceptables, dans la fabrication d'un médicament à utiliser dans l'inhibition de la thrombine ou la prévention du thrombus ou de la formation d'embole chez un mammifère.

8. Utilisation d'un composé selon l'une quelconque des revendications 1 à 5, ou d'un de ses sels pharmaceutiquement acceptables, pour éviter la coagulation de sang entier stocké ou la coagulation d'autres échantillons biologiques contenant de la thrombine.
